Europäisches Patentamt

European Patent Office

Office européen des brevets

(11) Publication number: **0 243 670 B1**

(12) **EUROPEAN PATENT SPECIFICATION**

(45) Date of publication of patent specification: **22.07.92**

(21) Application number: **87104286.7**

(22) Date of filing: **15.09.81**

(60) Publication number of the earlier application in accordance with Art.76 EPC: **0 049 072**

The file contains technical information submitted after the application was filed and not included in this specification

(51) Int. Cl.⁵: **C07D 473/18**, C07D 251/16, C07D 239/47, C07D 239/54, C07D 239/553

(54) Purine and pyrimidine compounds and their use as anti-viral agents.

(30) Priority: **16.09.80 US 187631**

(43) Date of publication of application:
**04.11.87 Bulletin 87/45**

(45) Publication of the grant of the patent:
**22.07.92 Bulletin 92/30**

(84) Designated Contracting States:
**AT BE CH DE FR GB IT LI LU NL SE**

(56) References cited:
EP-A- 0 066 208
US-A- 4 146 715

TETRAHEDRON LETTERS, vol. 21, 1980, pages 327-330, Pergamon Press Ltd, Oxford, GB; K.K. OGILVIE et al.: "Ring open analogues of deoxynucleotides"

(73) Proprietor: **SYNTEX (U.S.A.) INC.**
**3401 Hillview Avenue**
**Palo Alto California 94303(US)**

(72) Inventor: **Ogilvie, Kelvin K.**
**54 Place de Bretagne**
**Candiac Province of Ouebec(CA)**

(74) Representative: **Barz, Peter, Dr. et al**
**Patentanwälte Dr. V. Schmied-Kowarzik**
**Dipl.-Ing. G. Dannenberg Dr. P. Weinhold Dr.**
**D. Gudel Dipl.-Ing. S. Schubert Dr. P. Barz**
**Siegfriedstrasse 8**
**W-8000 München 40(DE)**

Rank Xerox (UK) Business Services

## Description

This invention relates to purine and pyrimidine compounds and to the use of such compounds as anti-viral agents.

Nucleosides comprise a D-ribose or 2-deoxy-D-ribose sugar unit, chemically bonded to a purine or pyrimidine base selected from adenine, cytosine, guanine, thymine and uracil, via a nuclear nitrogen atom of the base. Since they are units of nucleic acids found naturally in living cells, it has been speculated previously that nucleosides and nucleotides and their analogues might have potential as chemotherapeutic agents. Any practical value they may have, however, is often greatly reduced by their ready deamination in vivo by deaminases. Studies have been conducted to determine the relationship between structure and activity for both substrates and inhibitors of adenosine deaminase, some of said studies involving ring-opened analogues of nucleosides.

To date, however, despite several promising reports of novel compounds, no such compounds have been produced and developed for chemotherapeutic use, with the possible exception of acycloguanosine which is one of the purine compounds, described in US-A-4146715, having the formula

$$Z\text{-}CHR^6\text{-}X\text{-}CHR^3\text{-}CHR^4R^5$$

wherein Z is a purine group; X is oxygen or sulphur; $R^3$ is H, alkyl, hydroxyalkyl, benzyloxyalkyl or phenyl; $R^4$ is H, hydroxy or alkyl; $R^5$ is H, OH, amino, alkyl, hydroxyalkyl, benzoyloxy, benzoyloxymethyl, benzyloxy, sulphamoyloxy, phosphate, acyloxy or substituted carbamoyl; and $R^6$ is H or alkyl; with the proviso that, when X is oxygen, $R^3$, $R^4$ and $R^6$ are H, and $R^5$ is H, OH or benzyloxy, Z is not adenine or 6-N-methyladenine.

Ogilvie et al, Tetrahedron Letters, 21 (1980) 327-330, disclose purine and pyrimidine compounds of the formula

$$Z\text{-}CH_2\text{-}O\text{-}CH(CH_2OR')\text{-}CH_2OR \qquad I$$

wherein Z is adenine or thymine and R and R' are chosen from H, monomethoxytrityl, t-butyldimethylsilyl and benzyl.

Novel 9-substituted-purine and 1-substituted-pyrimidine compounds according to the present invention are of formula I, wherein Z represents a uracil, 5-fluorouracil, cytosine, 5-azacytosine or $N^2$-acetylguanine group; and R and R' are independently selected from H, $C_{1-6}$ alkyl and phenyl-($C_{1-6}$ alkyl); and pharmaceutically-acceptable salts thereof; provided that when Z is $N^2$-acetylguanine, R and R' are not both hydrogen or benzyl.

For example, R and R' are independently selected from H and benzyl. Z is preferably 5-fluorouracil (in which case R and R' are preferably each H or benzyl), 5-azacytosine (in which case R and R' are preferably each H), cytosine (in which case R and R' are preferably each benzyl) or $N^2$-acetylguanine.

It will be appreciated that the compounds according to the present invention, like those disclosed by Ogilvie et al, are closely analogous in structure and groupings to naturally-occurring nucleosides and nucleotides. The essential chain arrangements and lengths are maintained. The appropriate O and OH functional groups, which in biological environments actively bind to biological centres, are maintained in their natural sequences and disposition relative to the base, but optionally modified with "protecting" groups. Indeed, the groups adjacent to the bases are so similar in chemical constitution to deoxyribose compounds that they can assume the essential conformation of the deoxyribose ring under appropriate conditions. The fundamental difference is that the compounds of the present invention, like those disclosed by Ogilvie et al, lack the structural rigidity of the carbohydrate ring, which renders them unpredictably different in properties and behaviour. Also, the C-4' position is not chiral, in compounds of formula I, so that stereoisomers do not arise. Each hydroxyl is primary. There can be no syn-anti isomerism about the glycosidic bond.

Compounds of formula I may be made by coupling the appropriately halogenated purine or pyrimidine base with the appropriate alkyl residue. The synthesis may be initiated by treating 1,3-dichloro-2-propanol with sodium benzylate under a nitrogen atmosphere, followed by s-trioxane and HCl, to prepare the chloromethoxy derivative, care being taken to remove excess water. This derivative of 1,3-dibenzyloxy-2-propanol may be coupled to the appropriately halogenated base, such as 6-chloropurine, in DMF using triethylamine as acid scavenger. Treatment of the chloro compound so formed with methanolic ammonia in a steel reaction bomb gives the (not claimed) 6-amino derivative. The product may be debenzylated to give a compound of formula I, e.g. with hydrogen over palladium oxide in methanol. Protecting groups, if desired, are put on by standard, known methods. Alternatively, halogenated alkyl residues may be coupled with halogenated or non-halogenated purine or pyrimidine base compounds.

The compounds of the present invention show anti-viral activity, accompanied by low cell toxicity, rendering them potentially useful in therapeutic compositions to combat specific viral invaders of living mammalian cells.

Compounds according to the present invention may be administered to a patient parenterally, interthecally applied topically as ointment, cream, aerosol or powder, or even on occasion given as eye or nose drops or orally. In general, methods of administration and dosage formulations thereof follow the known, published methods used with known anti-viral drugs such as acycloguanosine, adenine arabinoside and 2′-deoxy-5-iodourine. Effective unit doses for administration of the compositions interthecally or parenterally, calculated as free base, are suitably in the range 0.1 to 100 mg per kg mammal body weight, and most preferably about 5 mg per kg, on the basis of a dosage administered 2 to 4 times per day.

Orally-administrable compositions are preferably in fine powder or granule form, with diluting and/or dispersing and/or surface-active agents, e.g. in water or in a syrup dispersion, or as tablets or capsules. Solutions of the compounds in distilled water or saline, e.g. isotonic solutions and optionally buffered with phosphate, of concentration 1 to 20%, preferably 2 to 15%, and most preferably around 10%, are suitable for parenteral or interthecal administration. Ointments (topical or cream) may be compounded for treatment of external infections, e.g. with an oil-in-water cream base, in a concentration of from 0.1 to 10%, preferably up to 3%, most preferably about 1%, w/v active ingredient. They may be compounded with paraffin oil, with petrolatum to form an emulsion optionally with a surfactant for stabilising purposes, or with dimethyl sulfoxide.

The invention is further illustrated by the following non-limitative Examples.

Example 1    1-[[Bis(benzyloxymethyl)methoxy]methyl]-5-fluorouracil (Dibenzyl-5FU*)

5-Fluorouracil (1.0 g, 0.0077 mole) and some crystals of ammonium sulfate were suspended in 1,1,1,3,3,3-hexamethyldisilazane (HMDS) (15 g) and brought to reflux with stirring. After 50 minutes, the base had dissolved and the excess HMDS was evaporated under reduced pressure to yield syrup of silyl-protected base (structure not determined). The syrup was dissolved in 1,2-dichloroethane (80 ml), and anhydrous stannic chloride (0.4 ml) was added.    1,3-Dibenzyloxy-2-chloromethoxypropane (0.007 mmole) from a stock solution was added, and the solution was stoppered and allowed to stand at room temperature overnight. The reaction mixture was shaken with aqueous sodium bicarbonate, and the phases were separated. The aqueous phase was extracted with chloroform. The combined organic phases were washed once with water, dried with anhydrous sodium sulfate and evaporated under reduced pressure, to yield 4.57 g of material. The nmr spectrum of the crude material suggested that the proportion of desired compound in the mixture was 88%. The material was mixed with 15 g silica (Fisher-S-662) and applied to a silica column (93 × 2.0 cm). The column was eluted with 1% methanol in chloroform (250 ml), 3% methanol in chloroform (200 ml), and 5% methanol in chloroform (700 ml). When coloured material began to appear, fractions were collected; the first 16 test tubes contained the desired material. The fractions were individually evaporated to syrups which, on standing overnight, crystallised. Some methanol was added, the crystals were broken up, and the solvent was removed with a Pasteur pipette. The crystals were washed once with methanol, to yield 0.886 g of crystals and 2.231 g of mother liquor residues. The residues were crystallised from carbon tetrachloride, and the resultant mother liquor residues were applied to preparative tlc plates and eluted with 5% methanol in chloroform. Eventually, 1.81 g (0.0044 mole, 62%) of crystalline dibenzyl-5FU* was obtained. An analytical sample was obtained by recrystallising the above material (tlc still showed an impurity) from a minimum of hot ethanol. The crystals gave: mp. 84 to 86 C and UV (EtOH) spectrum with $\lambda_{max}$265 nm.

Example 2    5-Fluoro-1-[[bis(hydroxymethyl)methoxy]methyl]uracil (5FU*)

Dibenzyl-5FU* prepared as described in Example 1, (0.0656 g, 0.00158 mole) was dissolved in ethanol (26 ml). Fresh palladium black (10 ml) was added, followed by cyclohexene (13 ml). After a half-hour, tlc showed that most of the starting material was gone but there was still a fair amount of monobenzyl compound present. After 5 hours, tlc showed that the reaction was complete; the mixture was filtered, and the Pd black was washed with ethanol. The solution was evaporated under reduced pressure, to yield 0.413 g of syrup which crystallised after the addition of some methanol. The sample was dissolved in 3 ml of hot ethanol, and then the volume was reduced to 1 ml by blowing with nitrogen. The resultant crystals (contained in a Greg tube) were washed 3 times with ethanol, and the residual solvent was removed by centrifugation to yield 0.185 g (0.00079 mole, 50%) of crystals. The mother liquor yielded another 0.089 g (0.00038 mole, 24%) of crystals. A second recrystallisation gave mp. 126 to 128°C and a UV spectrum (EtOH) $\lambda_{max}$266 nm. The nmr spectrum in $CD_3OD$ and TMS gave: 3.58 (m, 5 H, -$CH_2$CHCH$_2$-), 5.28 (s, 2 H, O$CH_2$N), 7.85 (d, I H, $J_{F6}$ = 6.0 Hz, H-6).

Example 3    5-Aza-1-[[1-bis(benzyloxymethyl)methoxy]methyl]cytosine (Dibenzyl-5-aza-C*)

5-Azacytosine (5.0 g, 0.0446 mole) and some ammonium sulfate (100 mg) were suspended in HMDS (40 ml) and brought to reflux with stirring. After 20 minutes, more ammonium sulfate was added, and 20 minutes later the mixture became clear. The excess HMDS was evaporated under reduced pressure to yield a white solid, silyl-protected 5-azacytosine, which was used without further purification. The solid was dissolved in DCE (100 ml), and anhydrous stannic chloride (3.5 ml) was added. Then 1,3-dibenzyloxy-2-chloromethoxypropane (0.040 mole) was added, and the solution was allowed to stand overnight at room temperature. The reaction solution was poured into aqueous bicarbonate, diluted with chloroform and shaken. The resultant precipitate was removed by filtration through Celite (registered Trade Mark). The phases were separated. The aqueous phase was extracted once with chloroform. The organic phases were combined, washed once with water, dried with anhydrous sodium sulfate, and evaporated to yield 15 g of syrup. The syrup was dissolved in chloroform (20 ml), and applied to a tlc silica column (14.5 × 6.5 cm). The column was first eluted with chloroform (450 ml). The solvent was changed to 5% methanol in chloroform and the collection of fractions (10 to 15 ml) was begun. Dibenzyl-5-aza-C* was found in three groupings of fractions which were: 40 to 42 (1.48 g), 43 to 62 (8.17 g) and 63 to 79 (0.42 g). The groupings were dissolved in 2.3 ml, 12 ml and 1.5 ml of hot ethanol respectively, and seeded. The second sample yielded 5.292 g of crystals, whereas the other two samples gave few crystals. Therefore the first and third sample were combined with the mother liquor of the second sample and applied to a short silica column (4.0 × 6.3 cm), and first eluted with chloroform (125 ml) and then with 5% methanol in chloroform when the collection of fractions (20 ml) was begun. The desired material appeared in fractions 7 to 8 (1.35 g) and 9 to 11 (1.9 g). The 1.9 g sample was dissolved in ethanol (3 ml) and 0.917 g of crystals resulted. The yield of compound was 6.209 g (0.0157 mole, 39%). The crystals (6.209 g) were augmented by crystals (1.461 g) from another experiment and recrystallised from ethanol (10 ml) to give 6.95 g of white crystals. A sample was recrystallised twice from ethanol and it gave: mp 120 to 122.5°C. The UV spectrum gave: $_{max}$ (EtOH) 228, 236, ($H_2O$) 241, (pH 1) 251, (pH 13) 250. The nmr spectrum ($CDCl_3$) gave: 3.52 (d, 4 H, J = 5.5 Hz, $CH_2CHCH_2$-), 4.02 (m, 1 H, -$CH_2CHCH_2$-), 4.45 (s, 4 H, 2 x $PhCH_2$-), 5.30 (s, 2 H, $OCH_2N$), 5.95 (bs, 1 H, HNH), 7.27 (m, 11 H, 2 x $PhCH_2$-, HNH), 8.07 (s, 1 H, H-6).

Example 4  5-Aza-1-[[bis(hydroxymethyl)methoxy]-methyl]cytosine (5-aza-C*)

Dibenzyl-5-aza-C* (6.432 g, 0.0162 mole) was dissolved in ethanol (200 ml, warmed). Used palladium oxide (8 g) was added followed by cyclohexene (100 ml). The stirred mixture was brought to reflux and, 3 hours later, tlc suggested that the reaction was slow. Therefore, fresh palladium oxide (2 g) was added and reflux was continued. After a total time of 22 hours, some material had precipitated out but the tlc still showed some starting material and the monobenzyl analogue. The material was filtered and the residue was washed with hot 95% ethanol until no more white precipitate was present. The filtrate and washing were combined and evaporated to yield about 4.5 g of material. The material was recrystallised from hot ethanol and some water (to help dissolution), to yield slightly greenish crystals (0.816 g, 0.00377 mole, 23.3%), mp 181 to 5°C; the filtrate was green. The filtrate was evaporated and the residue was dissolved in hot ethanol (3.5 ml) and seeded. Crystals did not form. The material was shaken with water and chloroform and the phases were separated. The aqueous phase was co-evaporated with ethanol, to yield 2 g of material. Crystallisation in the usual way was not successful. The tlc showed a number of components with the desired compound comprising about 30 to 40% of the mixture. The material was applied to 4 prep. tlc plates and developed with 50% methanol in chloroform. The desired band was eluted with 25% methanol in chloroform to yield 0.5 g of material. Crystallisation in the usual way yielded only a trace of crystals. The first crop of crystals was recrystallised from water (1 ml) and ethanol (7 ml) to give white crystals which gave: mp. 189.5 to 191°C. The desired compound gave UV spectrum: $_{max}$ (EtOH) 220 nm, ($H_2O$) 220, 245S, (pH 1) 250 (pH 13) 248. The nmr spectrum ($CD_3OD$ + 5 drops DMSO-d6 + TMS) gave: 3.43-3.83 (m, 5 H, -$CH_2CHCH_2$-), 5.37 (s, 2 H, $OCH_2N$), 8.27 (s, 1 H, H-6).

Example 5  1-[[Bis(benzyloxymethyl)methoxy]-methyl]cytosine (Dibenzyl-C*)

Cytosine (5.0 g, 0.045 mole) was suspended in 80 ml (60 g) HMDS, and several crystals of ammonium sulfate were added (modelled on G. Ritzmann & W. Pfleiderer, Chem. Ber. 106, 1401 (1973)). The stirred mixture was protected from moisture and refluxed until a clear solution was obtained. If a clear solution was not obtained after one half-hour of reflux the addition of more ammonium sulfate gave a clear solution after another 10 minutes of reflux. The clear hot solution was connected to a water aspirator and the excess HMDS was carefully removed on a hot water bath to yield a white solid which was used in the next step without purification.

The 2,4-bis(trimethylsilyl)cytosine was dissolved in dry DCE (200 ml) and stannic chloride (3.4 ml, 29.1 mole, anhydrous freshly distilled) was added. Then 40 g of stock solution of 1,3-dibenzyloxy-2-chloromethoxypropane (40 moles) was added and the yellow solution was allowed to stand overnight at room temperature (modelled on B.U. Niedballa & H. Verbruggen, Angew. Chem.).

Example 6    $N^2$-Acetyl-9-[[1-bis(benzyloxymethyl)-methoxy]methyl]guanine (Dibenzyl-N-acetyl-G*)

2-N-Acetylguanine (1.93 g, 10 mmoles) and ammonium sulfate (100 mg) were suspended in HMDS (20 ml). The stirred mixture was refluxed for 3 hours when it became clear. The excess HMDS was removed under reduced pressure on a hot water bath to yield a white solid, silyl-protected 2-N-acetylguanine, which was used without further purification. The white solid was dissolved in DCE (50 ml); 1,3-dibenzyloxy-2-chloromethoxypropane (5 mmoles) was added followed by freshly distilled anhydrous stannic chloride (1 ml). The solution was allowed to stand overnight at room temperature. The solution was poured into a mixture of aqueous sodium bicarbonate and chloroform and shaken. The mixture was filtered through Celite to remove the precipitate. The phases were separated and the aqueous phase was extracted once with chloroform.

The combined organic phases were washed with water, dried with anhydrous sodium sulfate, and evaporated under reduced pressure to yield 2.19 g of material. The reaction product was dissolved in chloroform (5 ml) and applied to a tlc silica column (9 x 6.5 cm). The column was first eluted with chloroform (60 ml) and then the solvent was changed to 2% methanol in chloroform. Three reasonably pure fractions were obtained from test tubes 15 (0.047 g), 19 to 20 (0.148 g) and 23 to 27 (0.43 g). Each of the samples was crystallised with ethanol, and gave approx. 20 mg, 64 mg and 250 mg of material, respectively. On the basis of UV spectra, the 250 mg material was determined to the desired product. It was recrystallised from ethanol and gave mp. 142-144°C. The UV spectrum gave $\lambda_{max}$ (EtOH) 257,281 nm min. 227,272, $\lambda_{max}$ (H$_2$O) 259,278 (shoulder), (pH 1) 262, (pH 13) 263.

Testing and Evaluation

Herpes simplex virus (HSV) strains were grown and titrated at 36°C in human fetal fibroblasts derived from fetal tissues, and used for virus work before the tenth passage. Cells were grown and maintained in basal medium Eagle (BME; Auto-Pow, Flow Laboratories) supplemented with 0 112% sodium bicarbonate, 2 mM 1-glutamine, 2 mg neomycin per 100 ml and 5 to 20% calf serum. 5% BME, as described hereafter, indicated medium containing 5 ml of calf serum in a total volume of 100 ml.

The titer of the HSV strains is determined by a plaque titration method (Roizman & Roane, "Virology", 15, 75-79, 1961). Tissue culture dishes are seeded with cells and used for assays when approximately 75% monolayer. Volumes (0.2 ml) of logarithmic dilutions of the strain are inoculated on to each of two tissue culture dishes, and adsorbed for 1 hour with intermittent shaking; the inoculum is removed, and 2 ml of 5% BME containing 0.5% human immune serum globulin are added. After a 48 hour incubation period at 36°C in a 5% $CO_2$ atmosphere, the overlay medium is removed, and the cell sheets are stained with a 0.05% aqueous crystal violet solution. The number of plaques is counted, the duplicates averaged, and the number of plaque-forming units calculated.

The compounds are tested for activity against the herpes simplex strains using a stock solution of each compound freshly prepared by dissolving 1.2 mg in BME. Appropriate dilution of each compound are made in 5% BME containing 0.5% human immune serum globulin just before usage.

Tissue culture dishes (35 by 10 mm) with approximately 75% cell monolayer are inoculated with approximately 50 plaque-forming units of HSV per 0.2 ml, and the virus adsorbed for 1 hour, with intermittent shaking. After removal of the inoculum, 2 ml of 5% BME with 0.5% immune globulin and three-fold dilutions of the appropriate drug are added. One set of dishes receives no drug and is used as a control. After a 48-hour incubation period at 36°C in a 5% $CO_2$ atmosphere, the overlay medium is removed, the cells are strained as described above, and plaques are counted. The counts of replicate plates are averaged, and the number of plaques emerging in the presence of each drug dilution is calculated. The reduction in plaque size caused by the concentration of the drug, as compared with the relevant control, is also measured, visually. A reduction in plaque number indicates that the added compound is preventing the reproduction of the viral cells. A reduction in the area of the growing plaque indicates inhibition of plaque growth, i.e. inhibition of viral reproduction, caused by the drug.

The compound of Example 4, 5-aza-C*, showed high activity against HSV. It reduced plaque size by 25% at 5 $\mu$g/ml, by 50% at 30 $\mu$g/ml and by 75% at 110 $\mu$g/ml. It reduced plaque numbers by 25% at 8 $\mu$g/ml, by 50% at 30 $\mu$g/ml and by 75% at 140 $\mu$g/ml. It showed no evidence of toxicity at concentrations as high as 304 $\mu$g/ml.

The compound of Example 5, dibenzyl-C*, also exhibited activity against HSV.

The compound of Example 1, dibenzyl-5FU*, showed activity against HSV, reducing plaque size by 25% at 10 $\mu$g/ml and by 50% at 50 $\mu$g/ml, and reducing plaque numbers by 25% at 70 $\mu$g/ml and by 50% at 110 $\mu$g/ml. It showed no evidence of toxicity up to concentrations of 110 $\mu$g/ml.

The compound of Example 6, dibenzyl-N-acetyl-G*, showed some activity against HSV, reducing plaque size by 25% at 60 $\mu$g/ml and by 50% at 150 $\mu$g/ml, and reducing plaque numbers by 25% at 20 $\mu$g/ml, by 50% at 130 $\mu$g/ml and by 75% at 310 $\mu$g/ml.

## Claims
## Claims for the following Contracting States : BE, CH, DE, FR, GB, IT, LI, LU, NL, SE

1. A 9-substituted-purine or 1-substituted-pyrimidine compound of the formula

$$Z\text{-}CH_2\text{-}O\text{-}CH(CH_2OR')\text{-}CH_2OR \qquad I$$

wherein Z represents a uracil, 5-fluorouracil, cytosine, 5-azacytosine or $N^2$-acetylguanine group; and R and R' are independently selected from H, $C_{1-6}$ alkyl and phenyl ($C_{1-6}$ alkyl), with the proviso that, when Z is $N^2$-acetylguanine, R and R' are not both hydrogen or benzyl; or a pharmaceutically acceptable salt thereof.

2. A 1-substituted-pyrimidine compound according to claim 1, wherein Z is 5-fluorouracil.

3. A 1-substituted-pyrimidine compound according to claim 1, wherein Z is 5-azacytosine.

4. A 1-substituted-pyrimidine compound according to claim 1, wherein Z is cytosine.

5. A 9-substituted-purine compound according to claim 1, wherein Z is $N^2$-acetylguanine.

6. A compound according to any preceding claim, wherein R and R' are independently selected from H and benzyl.

7. A compound according to claim 2 or claim 3, wherein R and R' are each H.

8. A compound according to claim 2 or claim 4, wherein R and R' are each benzyl.

9. A compound according to any preceding claim for use in the treatment of viral infections in live mammalian cells.

## Claims for the following Contracting State : AT

1. Process for the preparation of a 9-substituted-purine or 1-substituted-pyrimidine compound of the formula

$$Z\text{-}CH_2\text{-}O\text{-}CH(CH_2OR')\text{-}CH_2OR \qquad (I)$$

wherein Z represents a uracil, 5-fluorouracil, cytosine, 5-azacytosine or $N^2$-acetylguanine group; and R and R' are independently selected from H, $C_{1-6}$ alkyl and phenyl ($C_{1-6}$ alkyl), with the proviso that, when Z is $N^2$-acetylguanine, R and R' are not both hydrogen or benzyl; or a pharmaceutically acceptable salt thereof; wherein

(a) the appropriately halogenated purine or pyrimidine base is coupled with the appropriate alkyl residue; or

(b) a halogenated alkyl residue is coupled with halogenated or non-halogenated purine or pyrimidine base compound; or

(c) a compound of formula (I) wherein R and R' are benzyl is debenzylated to afford a compound of formula (I) wherein R and R' are hydrogen.

2. Process according to claim 1, wherein a compound wherein Z is 5-fluorouracil is prepared.

3. Process according to claim 1, wherein a compound wherein Z is 5-azacytosine is prepared.

4. Process according to claim 1, wherein a compound wherein Z is cytosine is prepared.

5. Process according to claim 1, wherein a compound wherein Z is $N^2$-acetylguanine is prepared.

6. Process according to any preceding claim, wherein a compound wherein R and R' are independently selected from H and benzyl is prepared.

7. Process according to claim 2 or claim 3, wherein a compound wherein R and R' are each H is prepared.

8. Process according to claim 2 or claim 4, wherein a compound wherein R and R' are each benzyl is prepared.

9. Process according to any preceding claim wherein a compound for use in the treatment of viral infections in live mammalian cells is prepared.

**Revendications**
**Revendications pour les Etats contractants suivants : BE, CH, DE, FR, GB, IT, LI, LU, NL, SE**

1. Composé purique substitué en position 9 ou pyrimidique substitué en position 1, répondant à la formule

   $Z\text{-}CH_2\text{-}O\text{-}CH(CH_2OR')\text{-}CH_2OR$     (I)

   dans laquelle Z représente un groupe uracile, 5-fluorouracile, cytosine, 5-azacytosine ou $N^2$-acétylguanine ; et R et R' sont choisis indépendamment entre H, des groupes alkyle en $C_1$ à $C_6$ et phényl-(alkyle en $C_1$ à $C_6$) sous réserve que, lorsque Z représente un groupe $N^2$-acétylguanine, R et R' ne représentent pas l'un et l'autre l'hydrogène ou un groupe benzyle ; ou un de ses sels pharmaceutiquement acceptables.

2. Composé pyrimidique substitué en position 1 suivant la revendication 1, dans lequel Z représente un groupe 5-fluoro-uracile.

3. Composé pyrimidique substitué en position 1 suivant la revendication 1, dans lequel Z représente un groupe 5-azacytosine.

4. Composé pyrimidique substitué en position 1 suivant la revendication 1, dans lequel Z représente un groupe cytosine.

5. Composé purique substitué en position 9 suivant la revendication 1, dans lequel Z représente un groupe $N^2$-acétylguanine.

6. Composé suivant l'une quelconque des revendications précédentes, dans lequel R et R' sont choisis indépendamment entre H et un groupe benzyle.

7. Composé suivant la revendication 2 ou la revendication 3, dans lequel R et R' représentent chacun H.

8. Composé suivant la revendication 2 ou la revendication 4, dans lequel R et R' représentent chacun un groupe benzyle.

9. Composé suivant l'une quelconque des revendications précédentes, destiné à être utilisé dans le traitement d'infections virales dans des cellules vivantes de mammifères.

**Revendications pour l'Etat contractant suivant : AT**

1. Procédé de préparation d'un composé purique substitué en position 9 ou d'un composé pyrimidique substitué en position 1, répondant à la formule

   $Z\text{-}CH_2\text{-}O\text{-}CH(CH_2OR')\text{-}CH_2OR$     (I)

   dans laquelle Z représente un groupe uracile, 5-fluorouracile, cytosine, 5-azacytosine ou $N^2$-acétylguanine ; et R et R' sont choisis indépendamment entre H, des groupes alkyle en $C_1$ à $C_6$ et phényl-(alkyle en $C_1$ à $C_6$) sous réserve que, lorsque Z représente un groupe $N^2$-acétylguanine, R et R' ne représentent pas l'un et l'autre l'hydrogène ou un groupe benzyle ; ou d'un de ses sels pharmaceutiquement acceptables ; dans lequel
   
   (a) la base purique ou pyrimidique halogénée de manière appropriée est couplée aux résidus alkyle appropriés ; ou
   (b) un résidu alkyle halogéné est couplé à une base purique ou pyrimidique halogénée ou non halogénée ; ou bien
   (c) un composé de formule (I), dans laquelle R et R' représentent un groupe benzyle, est débenzylé, ce qui donne un composé de formule (I) dans laquelle R et R' représentent l'hydrogène.

2. Procédé suivant la revendication 1, dans lequel est préparé un composé dont le substituant Z représente un groupe 5-fluoro-uracile.

3. Procédé suivant la revendication 1, dans lequel est préparé un composé dont le substituant Z représente un groupe 5-azacytosine.

4. Procédé suivant la revendication 1, dans lequel est préparé un composé dont le substituant Z représente un groupe cytosine.

5. Procédé suivant la revendication 1, dans lequel est préparé un composé dont le substituant Z représente un groupe $N^2$-acétylguanine.

6. Procédé suivant l'une quelconque des revendications précédentes, dans lequel est préparé un composé dont les substituants R et R' sont choisis indépendamment entre H et un groupe benzyle.

7. Procédé suivant la revendication 2 ou la revendication 3, dans lequel est préparé un composé dont les substituants R et R' représentent chacun H.

8. Procédé suivant la revendication 2 ou la revendication 4, dans lequel est préparé un composé dont les substituants R et R' représentent chacun un groupe benzyle.

9.  Procédé suivant l'une quelconque des revendications précédentes, dans lequel est préparé un composé destiné à être utilisé dans le traitement d'infections virales dans des cellules vivantes de mammifères.

**Patentansprüche**
**Patentansprüche für folgende Vertragsstaaten : BE, CH, DE, FR, GB, IT, LI, LU, NL, SE**

1.  9-Substituierte-Purin- oder 1-substituierte-Pyrimidinverbindung der Formel

    $Z-CH_2-O-CH(CH_2OR')-CH_2OR$        I

    worin Z eine Uracil-, 5-Fluoruracil-, Cytosin-, 5-Azacytosin- oder $N^2$-Acetylguaningruppe darstellt; und R und R' unabhängig aus H, $C_{1-6}$-Alkyl und Phenyl($C_{1-6}$-alkyl) ausgewählt sind, mit der Maßgabe, daß, wenn Z $N^2$-Acetylguanin ist, R und R' nicht beide Wasserstoff oder Benzyl sind; oder ein pharmazeutisch annehmbares Salz davon.

2.  1-Substituierte-Pyrimidinverbindung gemäß Anspruch 1, worin Z 5-Fluoruracil ist.

3.  1-Substituierte-Pyrimidinverbindung gemäß Anspruch 1, worin Z 5-Azacytosin ist.

4.  1-Substituierte-Pyrimidinverbindung gemäß Anspruch 1, worin Z Cytosin ist.

5.  9-Substituierte-Purinverbindung gemäß Anspruch 1, worin Z $N^2$-Acetylguanin ist.

6.  Verbindung gemäß irgendeinem der vorangehenden Ansprüche, worin R und R' unabhängig aus H und Benzyl ausgewählt sind.

7.  Verbindung gemäß Anspruch 2 oder Anspruch 3, worin R und R' beide H sind.

8.  Verbindung gemäß Anspruch 2 oder Anspruch 4, worin und R' beide Benzyl sind.

9.  Verbindung gemäß irgendeinem der vorangehenden Ansprüche zur Verwendung bei der Behandlung von Virusinfektionen in lebenden Säugerzellen.

**Patentansprüche für folgenden Vertragsstaat : AT**

1.  Verfahren zur Herstellung einer 9-substituierten-Purin- oder 1-substituierten-Pyrimidinverbindung der Formel

    $Z-CH_2-O-CH(CH_2OR')-CH_2OR$        (I)

    worin Z eine Uracil-, 5-Fluoruracil-, Cytosin-, 5-Azacytosin- oder $N^2$-Acetylguaningruppe darstellt; und R und R' unabhängig aus H, $C_{1-6}$-Alkyl und Phenyl-($C_{1-6}$-alkyl) ausgewählt sind, mit der Maßgabe, daß, wenn Z $N^2$-Acetylguanin ist, R und R' nicht beide Wasserstoff oder Benzyl sind; oder eines pharmazeutisch annehmbaren Salzes davon; worin

    (a) die geeignet halogenierte Purin- oder Pyrimidinbase mit dem geeigneten Alkylrest gekuppelt wird; oder
    (b) ein halogenierter Alkylrest mit halogenierter oder nicht-halogenierter Purin- oder Pyrimidinbase-Verbindung gekuppelt wird; oder
    (c) eine Verbindung der Formel (I), worin R und R' Benzyl sind, debenzyliert wird, um eine Verbindung der Formel (I), worin R und R' Wasserstoff sind, zu liefern.

2.  Verfahren gemäß Anspruch 1, worin eine Verbindung hergestellt wird, in der Z 5-Fluoruracil ist.

3.  Verfahren gemäß Anspruch 1, worin eine Verbindung hergestellt wird, in der Z 5-Azacytosin ist.

4.  Verfahren gemäß Anspruch 1, worin eine Verbindung hergestellt wird, in der Z Cytosin ist.

5.  Verfahren gemäß Anspruch 1, worin eine Verbindung hergestellt wird, in der Z $N^2$-Acetylguanin ist.

6.  Verfahren gemäß irgendeinem der vorangehenden Ansprüche, worin eine Verbindung hergestellt wird, in der R und R' unabhängig aus H und Benzyl ausgewählt sind.

7.  Verfahren gemäß Anspruch 2 oder Anspruch 3, worin eine Verbindung hergestellt wird, in der R und R' beide H sind.

8.  Verfahren gemäß Anspruch 2 oder Anspruch 4, worin eine Verbindung hergestellt wird, in der R und R' beide Benzyl sind.

9.  Verfahren gemäß irgendeinem der vorangehenden Ansprüche, worin eine Verbindung zur Verwendung bei der Behandlung von Virusinfektionen in lebenden Säugerzellen hergestellt wird.